# EUROPEAN PATENT SPECIFICATION

(11) **EP 3 251 533 B1**
(45) Date of publication and mention of the grant of the patent: **17.10.2018**
(21) Application number: 16172547.8
(22) Date of filing: 01.06.2016
(51) Int. Cl.: A41D 19/00, B29C 70/70, B32B 33/00, G01N 33/52, G09F 3/00, B29D 99/00, A61B 42/00, A61B 42/30, A61B 90/00, G09F 3/02

(54) **PROTECTIVE GLOVE WITH INDICATOR OF REMAINING LIFETIME**
SCHUTZHANDSCHUH MIT INDIKATOR DER VERBLEIBENDEN LEBENSDAUER
GANT DE PROTECTION AVEC INDICATEUR DE DURÉE DE VIE RESTANTE

(43) Date of publication of application: 06.12.2017
(73) Proprietor: Honeywell International Inc., Morris Plains, NJ 07950 (US)
(72) Inventor: COBIANU, Cornel, Morris Plains, NJ New Jersey 07950 (US); STRATULAT, Alisa, Morris Plains, NJ New Jersey 07950 (US); SERBAN, Bogdan, Morris Plains, NJ New Jersey 07950 (US); BUIU, Octavian, Morris Plains, NJ New Jersey 07950 (US); PIESKER, Andrea, Morris Plains, NJ New Jersey 07950 (US); FARIN, Eric, Morris Plains, NJ New Jersey 07950 (US)
(74) Representative: Houghton, Mark Phillip

(56) References cited:
- WO-A1-94/02080
- WO-A1-2015/188221
- US-A1- 2011 287 553
- US-B1- 6 175 962

## Description

### Technical Field

The present disclosure relates to protective gloves having an indicator of a remaining lifetime.

### Background

Protective gloves may be used in many types of industries and/or applications, such as, for instance, petrochemical, chemical, food and beverage, and/or pharmaceutical, to provide protection against various chemical, mechanical, and/or electrical materials and/or conditions. Different types of protective gloves (e.g., gloves of different materials and/or thicknesses) may be used in different applications to provide protection against different materials and/or conditions. For example, the type of protective glove used may depend on the type of chemicals to which the glove will be exposed to and/or used to handle during its use.

Protective gloves must meet a number of safety standards, such as, for instance, mechanical, thermal, and/or chemical standards, in order to be considered safe to use. Further, protective gloves should meet various comfort standards so they are comfortable for their user to wear. The particular standards a protective glove may need to meet may depend on the application for which the glove is to be used (e.g., on the materials and/or conditions the glove will be used to protect against). For example, the safety standards a protective glove must meet may depend on the number and/or types of chemicals to which the glove will be exposed to and/or used to handle, as well as the amount of time for which the glove will be continuously exposed to the chemicals.

Protective gloves may have a particular service lifetime for which they will be able to provide protection for their user, which may also depend on the application for which the gloves are used. When the gloves reach the end of their lifetime, due to, for example, permeation of chemicals through the materials of the gloves, they may not be able to provide adequate protection for its user. Accordingly, in order to effectively and safely use protective gloves, the user should be aware of the remaining lifetime of the gloves (e.g., how much service time is left before the gloves will fail to provide protection) while using the gloves.

It may be difficult, however, for the user to be able to quickly and/or accurately determine the remaining lifetime of the protective gloves while using the gloves. For example, the manufacturer and/or supplier of a protective glove may provide a recommended service lifetime for the glove. However, it may be difficult for the user to accurately keep track of the amount of time the glove is being used. Further, the recommended service lifetime provided by the manufacturer and/or supplier may not be accurate (e.g., may not correspond to the actual amount of time for which the glove will be able to provide protection). For example, the recommended service lifetime may be unnecessarily conservative, and/or may be based on a different application (e.g., different materials and/or conditions) than the one for which the glove is actually used.

US6175962B1 discloses a puncture resistant glove that includes an outer layer and inner layer of flexible plastic material, and a chemical mixture comprising cornstarch and an iodinized material between the inner and outer layer. It discloses that if a fluid passes through the outer layer and reaches the chemical mixture by passing through a perforation or puncture in the glove, the cornstarch and iodinized material will mix and cause a bright colored spot to appear.

WO2015188221A1 discloses an industrial glove that includes an exterior elastomeric layer, an indicating layer, and an interior elastomeric layer. It discloses that the indicating layer can include a colored dye that is organic solvent soluble, and that when the colored dye of the indicating layer comes into contact with chemicals, the indicating layer changes color.

### Brief Description of the Drawings

Figures 1A and 1B illustrate a protective glove with an indicator of a remaining lifetime in accordance with one or more embodiments of the present disclosure.
Figure 2 illustrates a protective glove with an indicator of a remaining lifetime in accordance with one or more embodiments of the present disclosure.
Figure 3 illustrates a protective glove with a number of remaining lifetime indicators in accordance with one or more embodiments of the present disclosure.
Figure 4 illustrates a method of forming a protective glove in accordance with one or more embodiments of the present disclosure.

### Detailed Description

Protective gloves with an indicator of a remaining lifetime are described herein. The present invention discloses a protective glove comprising a first layer of a plastic material having nanopores, a liquid soluble colorimetric material formed on the first layer of the plastic material, wherein the liquid soluble colorimetric material includes a colorant and is configured to provide an indicator of a remaining lifetime of the protective glove by dissolving upon being contacted by a liquid that has passed through the first layer of the plastic material by permeating through said nanopores in the first layer of the plastic material; and releasing, upon being dissolved, the colorant such that the colorant diffuses back through the first layer of the plastic material to an outer surface of the first layer of the plastic material of the glove and changes a color of the outer surface of the glove, and a second layer of the plastic material formed on the liquid soluble colorimetric material. A method of forming a protective glove according to the present invention is described in independent claim 9.

Protective gloves in accordance with the present disclosure can provide an indicator to their user (e.g., wearer) of their remaining lifetime, so that the user can effectively and safely use the gloves throughout their entire lifetime, until they are about to fail. For example, embodiments of the present disclosure can make it easy for the user to quickly and accurately determine the remaining lifetime of the gloves (e.g., how much service time is left before the gloves will fail to provide protection for the user). For instance, the user may not have to keep track of the amount of time the gloves have been used to know when they are approaching the end of their service lives. Further, the remaining lifetime indication provided in accordance with the present disclosure may correspond to the application(s) (e.g., materials and/or conditions) for which the gloves are actually used, and as such may be more accurate than the recommend service lifetime provided by the manufacturer and/or supplier of the gloves.

In the following detailed description, reference is made to the accompanying drawings that form a part hereof. The drawings show by way of illustration how one or more embodiments of the disclosure may be practiced.

These embodiments are described in sufficient detail to enable those of ordinary skill in the art to practice one or more embodiments of this disclosure. It is to be understood that other embodiments may be utilized and that process, and/or structural changes may be made without departing from the scope of the present invention according to claims 1-13.

The figures herein follow a numbering convention in which the first digit or digits correspond to the drawing figure number and the remaining digits identify an element or component in the drawing. Similar elements or components between different figures may be identified by the use of similar digits.

The proportion and the relative scale of the elements provided in the figures are intended to illustrate the embodiments of the present disclosure, and should not be taken in a limiting sense.

As used herein, "a" or "a number of" something can refer to one or more such things. For example, "a number of colorants" can refer to one or more colorants.

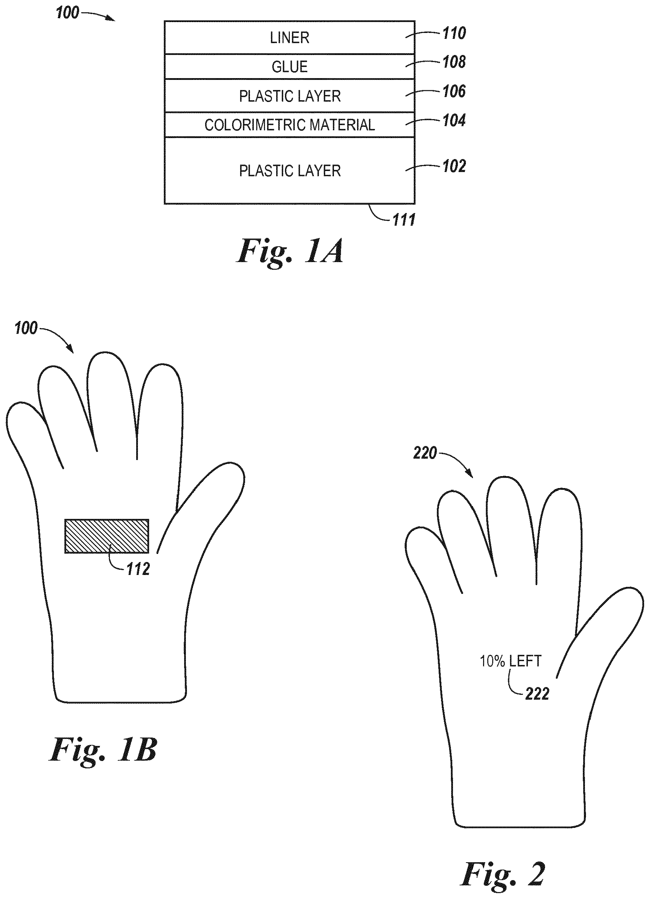

Figures 1A and 1B illustrate a protective glove 100 with an indicator 112 of a remaining lifetime in accordance with one or more embodiments of the present disclosure. For example, Figure 1A illustrates a schematic cross-sectional view of a portion of protective glove 100, and Figure 1B illustrates a top view of protective glove 100. The bottom of the portion of glove 100 illustrated in Figure 1A (e.g., layer 102) is the outer layer of glove 100, and the top of the portion of glove 100 illustrated in Figure 1A (e.g., liner 110) is the inner layer of glove 100 that is in direct contact with the skin of the user while the user is wearing the glove.

As shown in Figure 1A, protective glove 100 can include a first layer 102 of a plastic material. The plastic material can include, for example, a latex material (e.g., protective glove 100 can be a latex glove). However, embodiments of the present disclosure are not limited to a particular type of plastic material for protective glove 100. For example, in some embodiments, the plastic material can include a nitrile material, a butyl material, or a neoprene material, among other types of plastic materials.

As shown in Figure 1A, protective glove 100 can include a liquid soluble colorimetric material 104, such as, for instance, a water soluble colorimetric film, formed on the first layer 102 of the plastic material. Further, protective glove 100 can include a second layer 106 of the plastic material (e.g., the same type of plastic material included in first layer 102) formed on colorimetric material 104. That is, colorimetric material 104 can be formed (e.g., embedded) between layers 102 and 106 of the plastic material of protective glove 100, as illustrated in Figure 1A.

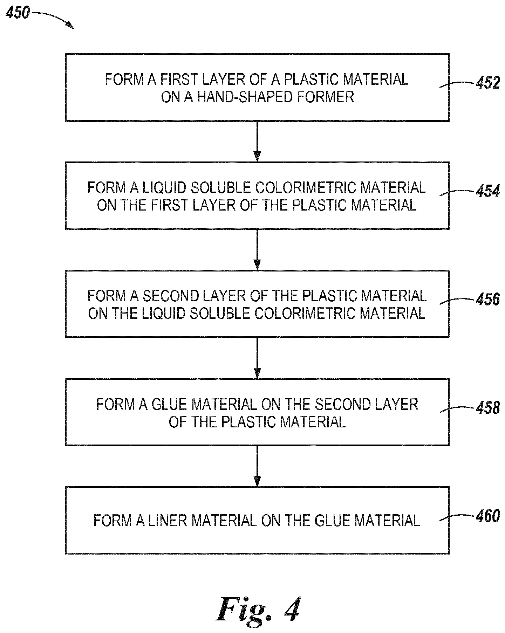

The processes by which colorimetric material 104 and layers 102 and 106 of the plastic material can be formed (e.g., fabricated) will be further described herein (e.g., in connection with Figure 4). Further, in the example illustrated in Figures 1A and 1B, colorimetric material 104 has been formed in a solid, contiguous shape (e.g., a rectangle). However, embodiments of the present disclosure are not so limited, as will be further described herein (e.g., in connection with Figure 2).

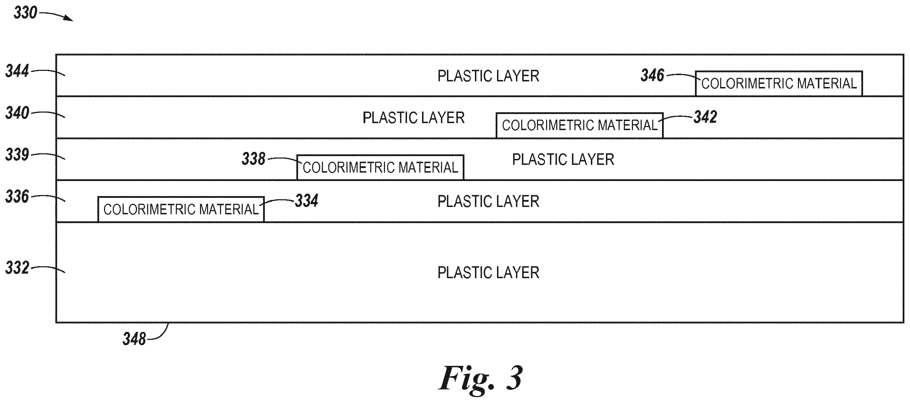

Further, although the embodiment illustrated in Figure 1A includes one (e.g., a single) liquid soluble colorimetric material between layers 102 and 106 of the plastic material, embodiments of the present disclosure are not so limited. For instance, protective gloves in accordance with some embodiments of the present disclosure can include more than one (e.g., multiple) colorimetric materials formed between the plastic material of the glove. An example of such a glove will be further described herein (e.g., in connection with Figure 3).

Liquid soluble colorimetric material 104 can provide an indicator of the remaining lifetime of protective glove 100. For example, colorimetric material 104 can provide an indicator to the user (e.g., wearer) of glove 100 of how much service time glove 100 has left before it will fail to provide protection for the user.

For example, liquid soluble colorimetric material 104 can include a number of colorants (e.g., dyes), such as, for instance, a number of small molecule pH indicators. Colorimetric material 104 can provide the indicator of the remaining lifetime of protective glove 100 by dissolving upon being contacted by a liquid, such as, for instance, a liquid (e.g., water) and/or vapor based solution, that has passed through (e.g., filled) layer 102 of the plastic material. Upon being dissolved, colorimetric material 104 can release the colorant(s) such that the colorant(s) diffuses much faster back through liquid (e.g., water) filled pores of layer 102 of the plastic material to the surface of glove 100 and changes the color of a portion of the surface of glove 100 that is visible to the user while the glove is being worn by the user.

An example of such an indicator (e.g., indicator 112) being provided (e.g., visible) to the user of glove 100 is illustrated in Figure 1B. In the example illustrated in Figure 1B, indicator 112 is located in the center area of glove 100, as that area is generally the area of the glove most frequently exposed to the liquid during use.

The liquid that passes through layer 102 of the plastic material may be and/or include the material(s) which protective glove 100 is providing protection against during its use. For example, the liquid may be and/or include the chemicals to which the glove has been exposed to and/or used to handle while being worn by the user.

The liquid may pass through layer 102 of the plastic material by, for example, permeating through the nanopores in layer 102 of the plastic material, and the colorant(s) can diffuse back through the liquid filled nanopores of layer 102. However, the molecular size of the colorant(s) may vary within a large range, depending on the type(s) of the colorant(s) used. For instance, the molecular size of a pH indicator of the colorant can be smaller than the molecular size of another pH indicator. On the other hand, the diffusion of the colorant(s) back though the liquid filled nanopores may depend on its molecular size, and this diffusion can be much faster than the permeation of an aqueous solution through a solid plastic material. As such, the colorant(s) can diffuse back through layer 102 of the plastic material at a faster rate than the rate at which the liquid can pass (e.g., forward) through layer 106 of the plastic material. Accordingly, the colorant(s) will reach the outer surface 111 of layer 102 of protective glove 100 and provide the indicator to the user well before the liquid (e.g., the chemicals in the liquid) can permeate through the nanopores in layer 106 of the plastic material.

The remaining lifetime of protective glove 100 indicated by colorimetric material 104 (e.g., by the colorant that has diffused to the surface of the glove) can correspond to the thickness of layer 106 of the plastic material, and its associated permeation time. As an example, the remaining lifetime of protective glove 100 indicated by colorimetric material 104 may be 10% of the total lifetime of the glove (e.g., the colorant diffusing to the outer surface 111 of layer 102 of the glove 100 and changing the color of a portion of the surface of the glove may indicate that the glove has 10% of its lifetime remaining). In such an example, layer 102 would include 90% of the total thickness of the plastic material of protective glove 100, and layer 106 would include the remaining 10% of the total thickness of the plastic material. However, embodiments of the present disclosure are not limited to this example.

The number of colorants in colorimetric material 104 can include, for example, a phenolphthalein (PPT) pH indicator material, a methyl red (MR) pH indicator material, a neutral red (NR) pH indicator material, and/or a methyl orange (MO) pH indicator material. For instance, in some embodiments colorimetric material 104 can include a PPT pH indicator material, in some embodiments colorimetric material 104 can include PPT and MR pH indicator materials, and in some embodiments colorimetric material 104 can include PPT and NR pH indicator materials. However, embodiments of the present disclosure are not limited to these particular examples.

PPT, MR, NR, and MO pH indicator materials can each have a small molecular size (e.g., smaller than the molecular size of other pH indicators such as, for instance, bromophenol blue, which has a molar mass of 670 grams/mol). For example, PPT, MR, NR, and MO have molar masses of 318 grams/mol, 269 grams/mol, 289 grams/mol, and 327 grams/mol, respectively. As such, these pH indicator materials can diffuse back through layer 102 of the plastic material at a fast rate and reach the outer surface 111 of layer 102 of protective glove 100 to provide the remaining lifetime indicator to the user well before the liquid can pass through layer 106 of the plastic material, as previously described herein.

Each respective colorant (e.g., each respective pH material) in colorimetric material 104 can be an indicator material for a different pH range. As such, the color change that occurs at the surface of protective glove 100 (e.g., the color of indicator 112) can correspond to (e.g., be an indicator of) the pH of the liquid that has passed through layer 102 of the plastic material. For instance, the outer surface 111 of layer 102 of glove 100 may change to one color if the liquid is an acid, and may change to another color if the liquid is a base.

For example, a PPT pH indicator material may change to fuchsia for a pH between 10.0 and 13.0, an MR pH indicator material may be red for a pH below 4.4 and change to yellow for a pH above 6.2, an NR pH indicator material may be red for a pH below 6.8 and change to yellow for a pH above 8.0, and an MO pH indicator may be red for a pH below 3.1 and change to yellow for a pH above 4.4. As such, in embodiments in which colorimetric material 104 includes PPT and MR pH indicator materials or PPT and NR indicator materials, the surface portion of protective glove 100 (e.g., indicator 112) may be red if the liquid that has passed through layer 102 of the plastic material is an acid, and may be yellow to fuchsia if the liquid is a base.

In some embodiments, colorimetric material 104 can include a polyvinyl alcohol (PVA) material. Further, a plasticizer material, such as, for instance, polyethylene glycol (PEG) can be added to the PVA material to increase the elasticity of colorimetric material 104.

As shown in Figure 1A, protective glove 100 can include a glue material 108 formed on layer 106 of the plastic material, and a liner material 110 formed on glue material 108. Liner material 110 can be the material of protective glove 100 that contacts the skin of the user while the user is wearing the glove. The processes by which glue material 108 and liner material 110 can be formed (e.g., fabricated) will be further described herein (e.g., in connection with Figure 4).

Figure 2 illustrates a top view of a protective glove 220 with an indicator 222 of a remaining lifetime in accordance with one or more embodiments of the present disclosure. Protective glove 220 can be analogous to protective glove 100 previously described in connection with Figures 1A and 1B. For example, protective glove 220 can include a liquid soluble colorimetric material formed between a first layer and a second layer of plastic material and configured to provide an indicator of the remaining lifetime of protective glove 220, in a manner analogous to protective glove 100.

The colorimetric material of glove 220, however, has been formed in an alphanumeric text pattern (e.g., instead of a solid, contiguous shape). For example, in the embodiment illustrated in Figure 2, the colorimetric material has been formed in the alphanumeric text pattern of "10% LEFT". As such, the indicator of the remaining lifetime of protective glove 220 provided to the user of the glove (e.g., indicator 222 illustrated in Figure 2) is the alphanumeric text pattern (e.g., 10% LEFT), which indicates the remaining lifetime of the glove (e.g., 10% of the total lifetime of the glove). The process by which such a colorimetric material can be formed (e.g., fabricated) will be further described herein (e.g., in connection with Figure 4).

Figure 3 illustrates a schematic cross-sectional view of a portion of a protective glove 330 with a number of remaining lifetime indicators in accordance with one or more embodiments of the present disclosure. The embodiment illustrated in Figure 3 includes multiple (e.g., more than one) liquid soluble colorimetric materials that can each provide a different remaining lifetime indicator.

For example, as shown in Figure 3, protective glove 330 can include a first layer 332 of a plastic material. The plastic material can include, for example, a latex material, a nitrile material, a butyl material, or a neoprene material, in a manner analogous to the plastic material previously described in connection with Figure 1A.

As shown in Figure 3, protective glove 330 can include a first liquid soluble colorimetric material 334, such as, for instance, a water soluble colorimetric film, formed on the first layer 332 of the plastic material. Further, protective glove 330 can include an additional layer 336 of the plastic material (e.g., the same type of plastic material included in first layer 332) formed on colorimetric material 334 and first layer 332, as illustrated in Figure 3. Further, protective glove 330 can include an additional liquid soluble colorimetric material 338 formed on layer 336 of the plastic material, an additional layer 339 of the plastic material formed on colorimetric material 338 and layer 336, an additional liquid soluble colorimetric material 342 formed on layer 339 of the plastic material, an additional layer 340 of the plastic material formed on colorimetric material 342 and layer 339, an additional liquid soluble colorimetric material 346 formed on layer 340 of the plastic material, and an additional layer 344 of the plastic material formed on colorimetric material 346 and layer 344, as illustrated in Figure 3. That is, in the embodiment illustrated in Figure 3, protective glove 330 includes four liquid soluble colorimetric materials. However, embodiments of the present disclosure are not limited to a particular number of liquid soluble colorimetric materials.

The processes by which the colorimetric materials and layers of the plastic material can be formed (e.g., fabricated) will be further described herein (e.g., in connection with Figure 4). Further, although the colorimetric materials have been formed in a solid, contiguous shape (e.g., a rectangle) in the example illustrated in Figure 3, in some embodiments the colorimetric materials can be formed in an alphanumeric text pattern, as previously described in connection with Figure 2. Further, although not shown in Figure 3 for simplicity and so as not to obscure embodiments of the present disclosure, protective glove 330 can include a glue material formed on layer 344, and a liner material formed on the glue material, in a manner analogous to glove 100 described in connection with Figure 1A.

Each respective one of the liquid soluble colorimetric materials 334, 338, 342, and 346 can provide an indicator of a different remaining lifetime of protective glove 330. For example, each respective one of the colorimetric materials can provide a different indicator to the user (e.g., wearer) of glove 330 of how much service time glove 330 has left before it will fail to provide protection for the user. For instance, colorimetric material 334 can provide an indicator of a first remaining lifetime of glove 330, colorimetric material 338 can provide an indicator of a second remaining lifetime of glove 330 that is less than the first remaining lifetime, colorimetric material 342 can provide an indicator of a third remaining lifetime of glove 330 that is less than the second remaining lifetime, and colorimetric material 346 can provide an indicator of a fourth remaining lifetime of glove 330 that is less than the third remaining lifetime.

For example, each respective one of the liquid soluble colorimetric materials 334, 338, 342, and 346 can include a number of colorants (e.g., dyes), such as, for instance, a number of small molecule pH indicators, and a PVA material. Each respective colorimetric material can provide its indicator of the remaining lifetime of protective glove 330 by dissolving and releasing its respective colorant(s), in a manner analogous to that previously described in connection with Figures 1A and 1B. The number of colorants in each respective colorimetric material can include, for example, PPT, MR, NR, and/or MO pH indicator materials that can have a small molecular size, can rapidly diffuse back through the liquid filled layers of the plastic material, and can be an indicator material for a different pH range, in a manner analogous to that previously described in connection with Figures 1A and 1B.

As shown in Figure 3, each respective liquid soluble colorimetric material 334, 338, 342, and 346 can be located at a different position in protective glove 330. For instance, each respective colorimetric material can be located at a different thickness and/or depth within the layers of the plastic material in glove 330, as illustrated in Figure 3. Each different position (e.g., each different thickness and/or depth) can correspond to a different remaining lifetime of glove 330. As such, the remaining lifetime indicated by each respective colorimetric material 334, 338, 342, and 346 can correspond to its position (e.g. its thickness and/or depth) in glove 330.

As an example, the remaining lifetime of protective glove 330 indicated by colorimetric material 334 may be 75% of the total lifetime of the glove (e.g., the colorant of colorimetric material 334 diffusing to the outer surface 348 of layer 332 of the glove 330 may indicate that the glove has 75% of its lifetime remaining), the remaining lifetime of glove 330 indicated by colorimetric material 338 may be 50% of the total lifetime of the glove (e.g., the colorant of colorimetric material 338 diffusing to the outer surface 348 of layer 332 of the glove 330 may indicate that the glove has 50% of its lifetime remaining), the remaining lifetime of glove 330 indicated by colorimetric material 342 may be 25% of the total lifetime of the glove (e.g., the colorant of colorimetric material 342 diffusing to the outer surface 348 of layer 332 of the glove 330 may indicate that the glove has 25% of its lifetime remaining), and the remaining lifetime of glove 330 indicated by colorimetric material 346 may be 10% of the total lifetime of the glove (e.g., the colorant of colorimetric material 346 diffusing to the outer surface 348 of layer 332 of the glove 330 may indicate that the glove has 10% of its lifetime remaining). In such an example, layers 332, 336, and 339 would each include 25% of the total thickness of the plastic material of protective glove 330 (e.g., layers 332, 336, and 339 combined would include 75% of the total thickness of the plastic material), layer 340 would include 15% of the total thickness of the plastic material, and layer 344 would include the remaining 10% of the total thickness of the plastic material. However, embodiments of the present disclosure are not limited to this example.

Figure 4 illustrates a method 450 of forming (e.g. fabricating) a protective glove in accordance with one or more embodiments of the present disclosure. For instance, method 450 can be used to form protective gloves 100, 220, and/or 330 previously described in connection with Figures 1A-1B, 2, and 3, respectively.

At block 452, method 450 includes forming a first layer of a plastic material on a hand-shaped former. The first layer of the plastic material can be, for example, layer 102 previously described in connection with Figure 1A or layer 332 previously described in connection with Figure 3.

The first layer of the plastic material can be formed on the hand-shaped former using, for example, a dip coating process. For instance, the hand-shaped former can be dip coated into a coagulant agent based on calcium nitrate, and can then be dip coated into a bath of the plastic material to form a plastic film. The dip coated hand-shaped former can then be thermally treated to obtain a solid state of the first layer of the plastic material.

At block 454, method 450 includes forming a liquid soluble colorimetric material on the first layer of the plastic material. The liquid soluble colorimetric material can be, for example, colorimetric material 104 previously described in connection with Figure 1A or colorimetric material 334 previously described in connection with Figure 3.

In some embodiments, the colorimetric material can be formed on the first layer of the plastic material using a dip coating process. For example, the hand-shaped former having the first layer of the plastic material formed thereon at block 452 can be dip coated into a bath of a viscous slurry of the colorimetric material, and then thermally treated (e.g., annealed) to obtain a solid film of the colorimetric material. The viscous slurry of the colorimetric material can be formed, for example, by dissolving a PVA powder in warm water, dissolving the pH indicator material(s) to be included in the colorimetric material in ethanol and adding this solution to the PVA solution, adding a plasticizer (e.g. PEG) to this solution, and stirring to obtain a homogeneous and viscous solution of the colorimetric material.

In some embodiments, the colorimetric material can be formed on the first layer of the plastic material using a direct printing process. For example, the colorimetric material can be directly printed on a selected area of the hand-shaped former having the first layer of the plastic material formed thereon at block 452, and then thermally treated to obtain a solid colorimetric material. Such a direct printing process may be used, for example, in embodiments in which the colorimetric material is to be formed in an alphanumeric text pattern (e.g., the embodiment illustrated in Figure 2) and/or in embodiments in which multiple colorimetric materials are to be formed (e.g., the embodiment illustrated in Figure 3).

In embodiments in which multiple colorimetric materials are to be formed, the hand-shaped former can again be dip coated into the bath of the plastic material and thermally treated to obtain an additional layer of the plastic material, and an additional colorimetric material can be formed (e.g., directly printed) on a different area of the hand-shaped former and thermally treated in an analogous manner. This process can continue to be repeated until all the colorimetric materials have been formed.

At block 456, method 450 includes forming a second layer of the plastic material on the liquid soluble colorimetric material. The second layer of the plastic material can be, for example, layer 106 previously described in connection with Figure 1A. The second layer of the plastic material can be formed using, for example, a dip coating process, in a manner analogous to the dip coating process used to form the first layer of the plastic material.

At block 458, method 450 includes forming a glue material on the second layer of the plastic material. The glue material can be, for example, glue material 108 previously described in connection with Figure 1A.

The glue material can be formed on the second layer using, for example, a dip coating process. For instance, the hand-shaped former having the second layer of the plastic material formed thereon at block 456 can be dip coated into a glue bath. The glue can then be dried at a mild thermal treatment.

At block 460, method 450 includes forming (e.g., adding) a liner material on the glue material. The liner material can be, for example, liner material 110 previously described in connection with Figure 1A. The glued-on liner can be given a final treatment, and the completed glove can be removed from (e.g., peeled off) the hand-shaped former.

Although specific embodiments have been illustrated and described herein, those of ordinary skill in the art will appreciate that any arrangement calculated to achieve the same techniques can be substituted for the specific embodiments shown. This disclosure is intended to cover any and all adaptations or variations of various embodiments of the disclosure.

It is to be understood that the above description has been made in an illustrative fashion, and not a restrictive one. Combination of the above embodiments, and other embodiments not specifically described herein will be apparent to those of skill in the art upon reviewing the above description.

The scope of the various embodiments of the disclosure includes any other applications in which the above structures and methods are used. Therefore, the scope of various embodiments of the disclosure should be determined with reference to the appended claims, along with the full range of equivalents to which such claims are entitled.

In the foregoing Detailed Description, various features are grouped together in example embodiments illustrated in the figures for the purpose of streamlining the disclosure. This method of disclosure is not to be interpreted as reflecting an intention that the embodiments of the disclosure require more features than are expressly recited in each claim.

## Claims

1. A protective glove (100, 220), comprising:
a first layer (102) of a plastic material having nanopores;
a liquid soluble colorimetric material (104) formed on the first layer (102) of the plastic material, wherein the liquid soluble colorimetric material (104) includes a colorant and is configured to provide an indicator (112, 222) of a remaining lifetime of the protective glove (100, 220) by:
dissolving upon being contacted by a liquid that has passed through the first layer (102) of the plastic material by permeating through said nanopores in the first layer (102) of the plastic material; and
releasing, upon being dissolved, the colorant such that the colorant diffuses back through the first layer (102) of the plastic material to an outer surface (111) of the first layer (102) of the plastic material of the glove (100, 220) and changes a color of the outer surface (111) of the glove (100, 220); and
a second layer (106) of the plastic material formed on the liquid soluble colorimetric material (104).

2. The protective glove (100, 222) of claim 1, wherein the colorant diffuses back through the first layer (102) of the plastic material at a faster rate than the liquid passes through the second layer (106) of the plastic material.

3. The protective glove (100, 222) of claim 1, wherein the remaining lifetime of the glove (100, 222) corresponds to a thickness of the second layer (106) of the plastic material.

4. The protective glove (100, 222) of claim 1, wherein the colorant includes a phenolphthalein pH indicator material.

5. The protective glove (100, 222) of claim 1, wherein the liquid soluble colorimetric material (104) includes a polyvinyl alcohol material.

6. The protective glove (100, 222) of claim 1, wherein the remaining lifetime of the protective glove (100, 222) is 10% of the lifetime of the glove.

7. The protective glove (100, 222) of claim 1, wherein the colorant includes a methyl or neutral red pH indicator material.

8. The protective glove (100, 222) of claim 1, wherein the plastic material includes a latex material.

9. A method (450) of forming a protective glove (100, 222), comprising:
forming a first layer (102) of a plastic material on a hand-shaped former;
forming a liquid soluble colorimetric material (104) on the first layer (102) of the plastic material, wherein the liquid soluble colorimetric material (104) includes a colorant and is configured to provide an indicator (112, 222) of a remaining lifetime of the protective glove (100, 220) by:
dissolving upon being contacted by a liquid that has passed through the first layer (102) of the plastic material by permeating through nanopores in the first layer (102) of the plastic material; and
releasing, upon being dissolved, the colorant such that the colorant diffuses back through the first layer (102) of the plastic material to an outer surface (111) of the first layer (102) of the plastic material and changes a color of the outer surface (111) of the glove (100, 220); and
forming a second layer (106) of the plastic material on the liquid soluble colorimetric material (104).

10. The method (450) of claim 9, wherein the method (450) includes forming the first layer (102) of the plastic material, the liquid soluble colorimetric material (104), and the second layer (106) of the plastic material using a dip coating process.

11. The method (450) of claim 9, wherein the method (450) includes forming the liquid soluble colorimetric material (104) using a direct printing process.

12. The method (450) of claim 9, wherein the method (450) includes forming the liquid soluble colorimetric material (104) in an alphanumeric text pattern.

13. The method (450) of claim 9, wherein the method (450) includes:
forming a glue material (108) on the second layer (106) of the plastic material; and
forming a liner material (110) on the glue material (108).

## Patentansprüche

1. Schutzhandschuh (100, 220), umfassend:
eine erste Schicht (102) eines Kunststoffmaterials mit Nanoporen;
ein flüssigkeitslösliches kolorimetrisches Material (104), das auf der ersten Schicht (102) des Kunststoffmaterials gebildet ist, wobei das flüssigkeitslösliche kolorimetrische Material (104) einen Farbstoff enthält und dafür gestaltet ist, einen Indikator (112, 222) für die verbleibende Lebenszeit des Schutzhandschuhs (100, 220) bereitzustellen durch:
Auflösen bei Kontakt mit einer Flüssigkeit, die durch die erste Schicht (102) des Kunststoffmaterials getreten ist, indem sie durch die Nanoporen in der ersten Schicht (102) des Kunststoffmaterials getreten ist; und
nach dem Auflösen Freisetzen des Farbstoffs, so dass der Farbstoff durch die erste Schicht (102) des Kunststoffmaterials zu der Außenoberfläche (111) der ersten Schicht (102) des Kunststoffmaterials des Handschuhs (100, 220) zurückdiffundiert und die Farbe der Außenoberfläche (111) des Handschuhs (100, 220) verändert;
und
eine zweite Schicht (106) des Kunststoffmaterials, die auf dem flüssigkeitslöslichen kolorimetrischen Material (104) gebildet ist.

2. Schutzhandschuh (100, 222) gemäß Anspruch 1, wobei der Farbstoff mit einer schnelleren Rate durch die erste Schicht (102) des Kunststoffmaterials zurückdiffundiert als die Flüssigkeit durch die zweite Schicht (106) des Kunststoffmaterials tritt.

3. Schutzhandschuh (100, 222) gemäß Anspruch 1, wobei die verbleibende Lebenszeit des Handschuhs (100, 220) einer Dicke der zweiten Schicht (106) des Kunststoffmaterials entspricht.

4. Schutzhandschuh (100, 222) gemäß Anspruch 1, wobei der Farbstoff ein Phenolphthalein-pH-Indikatormaterial umfasst.

5. Schutzhandschuh (100, 222) gemäß Anspruch 1, wobei das flüssigkeitslösliche kolorimetrische Material (104) ein Polyvinylalkoholmaterial enthält.

6. Schutzhandschuh (100, 222) gemäß Anspruch 1, wobei die verbleibende Lebenszeit des Schutzhandschuhs (100, 220) 10 % der Lebenszeit des Handschuhs beträgt.

7. Schutzhandschuh (100, 222) gemäß Anspruch 1, wobei der Farbstoff ein Methyl- oder Neutralrot-pH-Indikatormaterial umfasst.

8. Schutzhandschuh (100, 222) gemäß Anspruch 1, wobei das Kunststoffmaterial ein Latexmaterial umfasst.

9. Verfahren (450) zur Herstellung eines Schutzhandschuhs (100, 222), umfassend:
Bilden einer ersten Schicht (102) eines Kunststoffmaterials auf einer handförmigen Formvorrichtung;
Bilden eines flüssigkeitslöslichen kolorimetrischen Materials (104) auf der ersten Schicht (102) des Kunststoffmaterials, wobei das flüssigkeitslösliche kolorimetrische Material (104) einen Farbstoff enthält und dafür gestaltet ist, einen Indikator (112, 222) für die verbleibende Lebenszeit des Schutzhandschuhs (100, 220) bereitzustellen durch:
Auflösen bei Kontakt mit einer Flüssigkeit, die durch die erste Schicht (102) des Kunststoffmaterials getreten ist, indem sie durch die Nanoporen in der ersten Schicht (102) des Kunststoffmaterials getreten ist; und
nach dem Auflösen Freisetzen des Farbstoffs, so dass der Farbstoff durch die erste Schicht (102) des Kunststoffmaterials zu der Außenoberfläche (111) der ersten Schicht (102) des Kunststoffmaterials zurückdiffundiert und die Farbe der Außenoberfläche (111) des Handschuhs (100, 220) verändert; und
Bilden einer zweiten Schicht (106) des Kunststoffmaterials auf dem flüssigkeitslöslichen kolorimetrischen Material (104).

10. Verfahren (450) gemäß Anspruch 9, wobei das Verfahren (450) Bilden der ersten Schicht (102) des Kunststoffmaterials, des flüssigkeitslöslichen kolorimetrischen Materials (104) und der zweiten Schicht (106) des Kunststoffmaterials durch ein Tauchbeschichtungsverfahren umfasst.

11. Verfahren (450) gemäß Anspruch 9, wobei das Verfahren (450) Bilden des flüssigkeitslöslichen kolorimetrischen Materials (104) unter Verwendung eines Direktdruckverfahrens umfasst.

12. Verfahren (450) gemäß Anspruch 9, wobei das Verfahren (450) Bilden des flüssigkeitslöslichen kolorimetrischen Materials (104) mit einer alphanumerischen Textstruktur umfasst.

13. Verfahren (450) gemäß Anspruch 9, wobei das Verfahren (450) umfasst:
Bilden eines Klebstoffmaterials (108) auf der zweiten Schicht (106) des Kunststoffmaterials; und
Bilden eines Auskleidungsmaterials (110) auf dem Klebstoffmaterial (108).

## Revendications

1. Gant de protection (100, 220), comprenant :
une première couche (102) d'une matière plastique comportant des nanopores ;
un matériau colorimétrique soluble dans un liquide (104) formé sur la première couche (102) de la matière plastique, le matériau colorimétrique soluble dans un liquide (104) comprenant un colorant et étant configuré pour fournir un indicateur (112, 222) d'une durée de vie restante du gant de protection (100, 220) par :
dissolution après entrée en contact avec un liquide qui a traversé la première couche (102) de la matière plastique par perméation à travers lesdits nanopores dans la première couche (102) de la matière plastique ; et
libération, après dissolution, du colorant, de telle sorte que le colorant repasse par diffusion à travers la première couche (102) de la matière plastique pour parvenir à une surface extérieure (111) de la première couche (102) de la matière plastique du gant (100, 220) et change une couleur de la surface extérieure (111) du gant (100, 220) ; et
une deuxième couche (106) de la matière plastique formée sur le matériau colorimétrique soluble dans un liquide (104).

2. Gant de protection (100, 222) selon la revendication 1, dans lequel le colorant repasse par diffusion à travers la première couche (102) de la matière plastique à une vitesse plus rapide que le passage du liquide à travers la deuxième couche (106) de la matière plastique.

3. Gant de protection (100, 222) selon la revendication 1, dans lequel la durée de vie restante du gant (100, 222) correspond à une épaisseur de la deuxième couche (106) de la matière plastique.

4. Gant de protection (100, 222) selon la revendication 1, dans lequel le colorant contient un matériau indicateur de pH de type phénolphtaléine.

5. Gant de protection (100, 222) selon la revendication 1, dans lequel le matériau colorimétrique soluble dans un liquide (104) contient un matériau alcool polyvinylique.

6. Gant de protection (100, 222) selon la revendication 1, dans lequel la durée de vie restante du gant de protection (100, 222) est de 10 % de la durée de vie du gant.

7. Gant de protection (100, 222) selon la revendication 1, dans lequel le colorant contient un matériau indicateur de pH de type méthyle ou rouge neutre.

8. Gant de protection (100, 222) selon la revendication 1, dans lequel la matière plastique contient un matériau latex.

9. Procédé (450) de formation d'un gant de protection (100, 222), comprenant :
la formation d'une première couche (102) d'une matière plastique sur une forme de main ;
la formation d'un matériau colorimétrique soluble dans un liquide (104) sur la première couche (102) de la matière plastique, le matériau colorimétrique soluble dans un liquide (104) comprenant un colorant et étant configuré pour fournir un indicateur (112, 222) d'une durée de vie restante du gant de protection (100, 220) par :
dissolution après entrée en contact avec un liquide qui a traversé la première couche (102) de la matière plastique par perméation à travers les nanopores dans la première couche (102) de la matière plastique ; et
libération, après dissolution, du colorant, de telle sorte que le colorant repasse par diffusion à travers la première couche (102) de la matière plastique pour parvenir à une surface extérieure (111) de la première couche (102) de la matière plastique et change une couleur de la surface extérieure (111) du gant (100, 220) ; et
formation d'une deuxième couche (106) de la matière plastique sur le matériau colorimétrique soluble dans un liquide (104).

10. Procédé (450) selon la revendication 9, le procédé (450) comprenant la formation de la première couche (102) de la matière plastique, du matériau colorimétrique soluble dans un liquide (104), et de la deuxième couche (106) de la matière plastique, en utilisant un procédé de revêtement par trempage.

11. Procédé (450) selon la revendication 9, le procédé (450) comprenant la formation du matériau colorimétrique soluble dans un liquide (104) en utilisant un procédé d'impression directe.

12. Procédé (450) selon la revendication 9, le procédé (450) comprenant la formation du matériau colorimétrique soluble dans un liquide (104) de façon à produire un motif sous forme de texte alphanumérique.

13. Procédé (450) selon la revendication 9, le procédé (450) comprenant :
la formation d'un matériau de type colle (108) sur la deuxième couche (106) de la matière plastique ; et
la formation d'un matériau de couverture (110) sur le matériau de type colle (108).
